# EUROPÄISCHE PATENTSCHRIFT

(11) **EP 1 622 612 B1**
(45) Veröffentlichungstag und Bekanntmachung des Hinweises auf die Patenterteilung: **12.03.2008**
(21) Anmeldenummer: 04729635.5
(22) Anmeldetag: 27.04.2004
(51) Int. Cl.: A61K 31/4184, A61K 31/41, A61K 9/20

(54) **PHARMAZEUTISCHE FORMULIERUNG DES TELMISARTAN NATRIUMSALZES**
PHARMACEUTICAL FORMULATION OF THE SODIUM SALT OF TELMISARTAN
FORMULATION PHARMACEUTIQUE DU SEL DE SODIUM TELMISARTAN

(30) Priorität: 30.04.2003 DE 10319450
(43) Veröffentlichungstag der Anmeldung: 08.02.2006
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: KOHLRAUSCH, Anja, 88400 BIBERACH (DE)
(86) Internationale Anmeldenummer: PCT/EP2004/004425
(87) Internationale Veröffentlichungsnummer: WO 2004/096215

(56) Entgegenhaltungen:
- EP-A- 0 502 314
- WO-A-03/037846

## Beschreibung

Die Erfindung betrifft eine Arzneimittelformulierung des kristallinen Natriumsalzes von 4'-[2-n-Propyl-4-methyl-6-(1-methylbenzimidazol-2-yl)benzimidazol-1 - ylmethyl]biphenyl-2-carbonsäure (Telmisartan), zusammen mit dem Diuretikum Hydrochlorothiazid, sowie Verfahren zu deren Herstellung.

### Hintergrund der Erfindung

Die Verbindung Telmisartan ist aus dem Europäischen Patent EP-502 314-B1 bekannt und weist die folgende chemische Struktur auf:

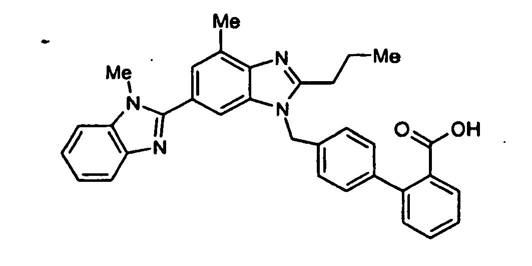

Telmisartan, sowie dessen physiologisch verträgliche Salze, besitzen wertvolle - pharmakologische Eigenschaften. Telmisartan stellt einen Angiotensin-Antagonisten, insbesondere einen Angiotensin-II-Antagonisten dar, der aufgrund seiner pharmakologischen Eigenschaften beispielsweise zur Behandlung der Hypertonie und Herzinsuffizienz, zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Neuropathie, des Glaukoms, von gastrointestinalen Erkrankungen sowie von Blasenerkrankungen Verwendung finden kann. Weitere mögliche Therapiegebiete sind der EP-502314-B1 sowie der WO 02/15891 zu entnehmen, auf die an dieser Stelle inhaltlich Bezug genommen wird.

Hydrochlorothiazide (HCTZ) ist ein Thiazid-Diuretikum das oral zur Behandlung von Ödemen und Bluthochdruck eingesetzt wird. Der chemische Name von HCTZ ist 6-Chloro-3,4-dihydro-2H-1,2,4-benzothiadiazin-7-sulfonamid-1,1-dioxid und die Verbindung wird durch folgende Strukturformel beschrieben:

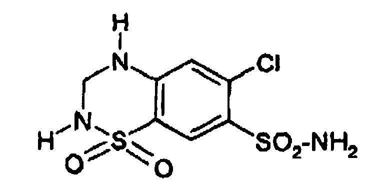

Telmisartan ist unter dem Handelsnamen Micardis^{®}, eine Kombination des Telmisartans mit Hydrochlorothiazid (HCTZ) unter dem Handelsnamen Micardis Plus^{®} im Handel erhältlich. Ausgehend von der freien Säure des Telmisartans werden diese Darreichungsformen über ein aufwendiges Sprühtrocknungsverfahren hergestellt. Aufgrund der geringen Löslichkeit der freien Säure sind weniger aufwendige Verfahren zur Herstellung einer alternativen Darreichungsform nur schwer realisierbar. Eine kristalline Form des Natriumsalzes von Telmisartan wird in WO 03/037876 beschrieben.

Es ist Aufgabe der vorliegenden Erfindung, Telmisartan in einer Form bereitzustellen, die die Darstellung einer Darreichungsform dieses Wirkstoffs in weniger aufwendiger Form erlaubt. Hierbei muß berücksichtigt werden, daß sich generell die Herstellung einer einen Arzneimittelwirkstoff enthaltenden Zusammensetzung von verschiedenen Parametern abhängig ist, die mit der Beschaffenheit des Wirkstoffes selbst verbunden sind. Ohne Einschränkung darauf sind Beispiele dieser Parameter die Wirkstabilität des Ausgangsstoffes unter verschiedenen Umgebungsbedingungen, die Stabilität im Verlauf der Herstellung der pharmazeutischen Formulierung sowie die Stabilität in den Endzusammensetzungen des Arzneimittels. Der zur Herstellung einer Arzneimittelzusammensetzung verwendete Wirkstoff sollte so rein wie möglich sein. Gleichzeitig muß seine Stabilität bei Langzeitlagerung unter verschiedenen Umgebungsbedingungen gewährleistet sein. Dies ist zwingend erforderlich, um zu verhindern, daß Arzneimittelzusammensetzungen Verwendung finden, in denen neben tatsächlichem Wirkstoff beispielsweise Abbauprodukte desselben enthalten sind. In einem solchen Fall könnte ein in späteren Darreichungsformen vorgefundener Gehalt an Wirkstoff niedriger sein als spezifiziert.

Ein weiterer für die Herstellung von festen Darreichungsformen bedeutsamer Aspekt ist darin zu sehen, daß für die pharmazeutische Qualität einer Arzneimittelformulierung eine möglichst stabile, kristalline Morphologie des Wirkstoffs gewährleistet sein sollte. Ist dies nicht der Fall, so kann es im Verlauf der Herstellung der Darreichungsform zu einer Veränderung der Morphologie des Wirkstoffes kommen. Eine solche Veränderung kann wiederum einen Einfluß auf die Reproduzierbarkeit des Herstellverfahrens haben und somit zu Endformulierungen führen, die den hohen Qualitätsanforderungen, die an Arzneimittelformulierungen zu richten sind, nicht genügen. Insofern ist generell zu berücksichtigen, dass jede Änderung des Feststoffzustandes eines Arzneimittels, welche dessen physikalische und chemische Stabilität verbessern kann, gegenüber weniger stabilen Formen desselben Arzneimittels einen erheblichen Vorteil ergibt.

Die Aufgabe der Erfindung besteht somit in der Bereitstellung einer neuen, pharmazeutischen Zusammensetzung enthaltend eine stabile Form des Telmisartans, die den vorstehend genannten hohen Anforderungen, die an einen Arzneimittelwirkstoff zu richten sind, genügen.

### Detaillierte Beschreibung der Erfindung

Es wurde überraschenderweise gefunden, daß Telmisartan in Form seines Natriumsalzes der Formel **1** 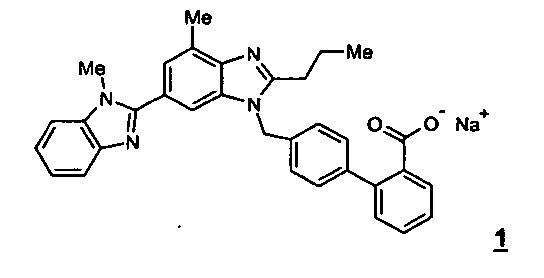 in kristalliner Form erhalten werden kann. Durch geeignete Wahl der Herstellungsbedingungen, kann dabei diejenige polymorphe Form des kristallinen Natriumsalzes, die den eingangs genannten Anforderungen genügt, in selektiver Art und Weise erhalten werden.

Diese kristalline Form des Telmisartan-Natriumsalzes ist gekennzeichnet durch einen Schmelzpunkt von T=245 ± 5°C (bestimmt über DSC=Differential Scanning Calorimetry; Heizrate: 10 K/min).

Nachstehende Tabelle 1 fasst im Rahmen einer spektroskopischen Analyse des Salzes erhaltene Daten zusammen:

**Tabelle 1:**

| **2 Θ [°]** | **d [Å]** | **rel. Intensität** | **2 Θ [°]** | **d [Å]** | **rel. Intensität** |
|---|---|---|---|---|---|
| | | **[%]** | | | **[%]** |
| 3,54 | 24,96 | 7 | 13,17 | 6,72 | 7 |
| 4,21 | 20,95 | 100 | 13,68 | 6,47 | 7 |
| 4,45 | 19,83 | 20 | 14,36 | 6,16 | 10 |
| 4,98 | 17,72 | 54 | 14,98 | 5,91 | 13 |
| 5,69 | 15,52 | 8 | 15,51 | 5,71 | 14 |
| 6,32 | 13,97 | 34 | 15,70 | 5,64 | 12 |
| 6,48 | 13,63 | 35 | 16,21 | 5,46 | 8 |
| 7,12 | 12,41 | 12 | 17,09. | 5,18 | 10 |
| 7,49 | 11,80 | 11 | 17,48 | 5,07 | 9 |
| 8,08 | 10,93 | 4 | 18,10 | 4,90 | 9 |
| 8,49 | 10,41 | 6 | 19,18 | 4,62 | 11 |
| 8,96 | 9,86 | 7 | 19,43 | 4,56 | 13 |
| 9,50 | 9,31 | 5 | 19,95 | 4,45 | 11 |
| 10,19 | 8,68 | 5 | 20,89 | 4,25 | 11 |
| 10,80 | 8,18 | 8 | 21,29 | 4,17 | 10 |
| 11,16 | 7.92 | 18 | 22,19 | 4,00 | 9 |
| 11,88 | 7,44 | 7 | 23,07 | 3,85 | 10 |
| 12,51 | 7,07 | 7 | 23,76 | 3,74 | 9 |
| 12,79 | 6,92 | 11 | 24,43 | 3,64 | 8 |

In vorstehender Tabelle steht der Wert "2 Θ [°]" für den Beugungswinkel in Grad und der Wert "d [Å]" für die bestimmten Gitterebenenabstände in Å.

Entsprechend den in Tabelle 1 dargestellten Befunden ist das kristalline Telmisartan-Natriumsalz, dadurch gekennzeichnet, daß es im Röntgenpulverdiagramm unter anderen die charakteristischen Werte d= 20,95 Å, 17,72 Å, 13,97 Å und 13,63 Å aufweist.

Die Röntgenpulverdiagramme wurden im Rahmen der vorliegenden Erfindung aufgenommen mittels eines Bruker D8 Advanced mit einem OED (= ortsempfindlicher Detektor) (CuK_{α} - Strahlung, λ = 1.5418 Å, 30 kV, 40 mA).

Das erfindungsgemäße kristalline Telimsartan-Natriumsalz kann auch in Form seiner Solvate und Hydrate vorliegen, bevorzugt in Form seiner Hydrate, besonders bevorzugt in Form seines Hemihydrats.

Ein weiterer Aspekt der vorliegenden Erfindung betrifft ein Herstellverfahren zur Darstellung des erfindungsgemäßen kristallinen Telmisartan-Natriumsalzes.
Als Ausgangsmaterial zur Darstellung von erfindungsgemäßem kristallinem Telmisartan-Natriumsalz kann die freie Säure Telmisartan dienen, die nach im Stand der Technik bekannten Verfahren (z.B. gemäß EP 502314 A1) erhalten werden kann.
Zur Darstellung des erfindungsgemäßen kristallinen Natriumsalzes wird die freie Säure des Telmisartans in einem geeigneten Lösemittel, vorzugsweise in einem organischen aprotischen Lösemittel, besonders bevorzugt in einem organischen, aprotischen und unpolaren Lösemittel aufgenommen. Als Lösemittel kommen erfindungsgemäß besonders bevorzugt in Betracht Toluol, Chloroform, Dichlormethan, Tetrahydrofuran, Diethylether, Diisopropylether, Methyl-tertiärbutylether, Aceton, Methylisobutylketon, Benzol oder Acetonitril, wobei Toluol Benzol und Methylisobutylketon besonders bevorzugt sind. Erfindungsgemäß von herausragender Bedeutung ist als Lösemittel Toluol.
Pro Gramm der freien Säure des Telimsartans gelangen in der Regel zwischen 0,5 und 5 ml, bevorzugt zwischen 1 und 3 ml, besonders bevorzugt zwischen 1,5 und 2,5 ml des vorstehend genannten Lösemittels zur Anwendung.
Zu dieser Lösung oder Suspension wird anschließend ein geeignetes Natriumsalz als Base zugesetzt. Als geeignete Natriumsalze kommen im Rahmen der vorliegenden Erfindung Natriumhydroxid, Natriumhydrid, Natriumcarbonat, Natriumhydrogencarbonat oder Natriumalkoholate in Betracht. Unter Natriumalkoholaten werden die Natriumsalze verstanden, die mit niederen Alkoholen, vorzugsweise mit Alkoholen ausgewählt aus der Gruppe bestehend aus Methanol, Ethanol, Isopropanol, n-Propanol, tert-Butanol, sec.-Butanol, iso-Butanol,n-Butanol und tert.-Amylalkohol, gebildet werden. Erfindungsgemäß von besonderem Interesse sind Natriumsalze ausgewählt aus der Gruppe bestehend aus Natriumhydroxid, Natriumhydrid, Natriumethanolat und Natriummethanolat, wobei das Natriumhydroxid und das Natriummethanolat von erfindungsgemäß herausragender Bedeutung ist. Die vorstehend genannten Natriumsalze können der Reaktionsmischung als Feststoffe zugesetzt werden. Im Falle des Natriumhydroxids ist die Zugabe in Form wässriger Lösungen allerdings bevorzugt. Hierbei werden besonders bevorzugt konzentrierte wässrige Lösungen des Natriumhydroxids verwendet. Beispielsweise kann Natronlage mit einer Konzentration von etwa 45 Gew.-% zum Einsatz gelangen.
Die Menge an einzusetzendem Natriumsalz ist naturgemäß abhängig von der Menge an eingesetzter freier Säure Telmisartan. Erfindungsgemäß müssen pro Mol Telmisartan wenigstens 1 Mol Natriumsalz zugesetzt werden. Eine Zugabe an Natriumsalz im Überschuß ist erfindungsgemäß möglich. Bevorzugt werden pro Mol eingesetzte Säure Telmisartan 1-2,5, bevorzugt 1-2, besonders bevorzugt 1-1,5 Mol Natriumsalz zugesetzt.
Wird als Natriumsalz Natriumhydroxid verwendet und dieses gemäß einer bevorzugten Ausführungsform des erfindungsgemäßen Verfahrens in Form wässeriger Lösungen zugesetzt, kann es gegebenenfalls hilfreich sein, ein mit Wasser mischbares organisches Lösemittel zuzusetzen. Dieses ist vorzugsweise ausgewählt aus der Gruppe bestehend aus Methanol, Ethanol, Isopropanol, Aceton, Tetrahydrofuran, tert.-Butanol, 2-Butanol, Butanol, Glycol, Ethyldiglycol, 1,3-Butandiol, 1,4-Butandiol, tert.-Amylalkohol, Acetonitril, Nitromethan, Formamid, Dimethylformamid, N-Methylpyrrolidinon, Dimethylsulfoxid, Dimethylacetamid, Nitroethan, Methoxy-2-Propanol, wobei den vorstehend genannten Alkoholen eine besondere Bedeutung zukommt. Besonders bevorzugt werden im Rahmen des erfindungsgemäßen Verfahrens Methanol oder Ethanol, und vor allem Ethanol verwendet. Pro Mol eingesetztes Telmisartan werden erfindungsgemäß bevorzugt zwischen 50 und 500 ml, besonders bevorzugt zwischen 100 und 400 ml, ferner bevorzugt zwischen 200 und 350 ml dieses Lösemittels verwendet. Anschließend kann die Reaktionsmischung erwärmt und damit das Fortschreiten der Reaktion beschleunigt werden. Bevorzugt wird die Reaktionsmischung unter guter Durchmischung auf eine Temperatur von >40°C, besonders bevorzugt auf über 60°C erwärmt. Die maximal wählbare Temperatur bestimmt sich natürlich durch die Siedetemperatur der verwendeten Lösemittel. Werden die erfindungsgemäß bevorzugten Lösemittel eingesetzt, wird bevorzugt auf über 70°C erwärmt. Diese Erwärmung wird in der Regel für einen Zeitraum von 15 Minuten bis 2 Stunden, bevorzugt zwischen 20 Minuten und einer Stunde durchgeführt. Anschließend wird die erhaltene Lösung filtriert und gegebenenfalls im Filter verbleibender Feststoff mit einem oder mehreren der vorstehend genannten Lösemittel nachgewaschen.

Zu einem auf eine Temperatur von >40°C, bevorzugt auf über 60°C, besonders bevorzugt bis zur Siedehitze erwärmten organischen Lösemittel, wird das gemäß vorstehend beschriebener Vorgehensweise erhaltene Filtrat langsam, vorzugsweise tropfenweise zugegeben. Als Lösemittel kommt an dieser Stelle vorzugsweise ein organisches aprotisches Lösemittel, besonders bevorzugt ein organisches, aprotisches und unpolares Lösemittel in Betracht. Als Lösemittel kommen erfindungsgemäß besonders bevorzugt in Betracht Toluol, Chloroform, Dichlormethan, Tetrahydrofuran, Diethylether, Diisopropylether, Methyl-tertiärbutylether, Aceton, Methylisobutylketon, Benzol oder Acetonitril, wobei Toluol, Benzol und Methylisobutylketon besonders bevorzugt sind. Erfindungsgemäß von herausragender Bedeutung ist an dieser Stelle das Lösemittel Toluol. Gleichzeitig werden während der Zugabe des Filtrats zu dem vorgelegten erwärmten Lösemittel bei einer bevorzugten Ausführungsform des Verfahrens Teile des Lösemittels (gegebenenfalls azeotrop) abdestilliert. Nach beendeter Zugabe wird gegebenenfalls weiteres Lösemittel (beispielhaft etwa 1-2 Drittel der bis zu diesem Zeitpunkt insgesamt eingesetzten Lösemittelmenge) destillativ entfernt.

Die so erhaltene konzentrierte Lösung wird abgekühlt, vorzugsweise auf Raumtemperatur, wobei Telmisartan-Natriumsalz auskristallisiert. Nach beendeter Kristallisation werden die Kristalle abgetrennt, gegebenenfalls mit dem eingangs genannten organischen Lösemittel gewaschen und abschließend getrocknet.

Das erfindungsgemäße kristalline Telmisartan-Natriumsalz ist auch ausgehend von den Säureadditionssalzen der Formel **2** 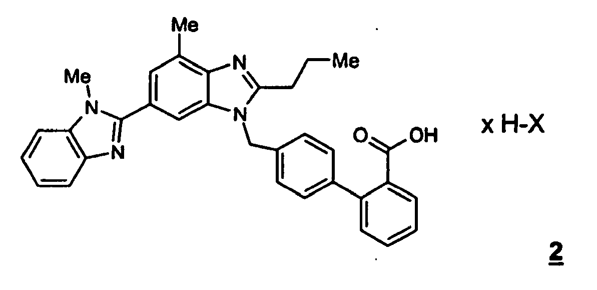 worin H-X für eine Säure ausgewählt aus der Gruppe bestehend aus Salzsäure, Bromwasserstoffsäure, Toluolsulfonsäure oder Methansulfonsäure steht, erhältlich. Von den vorstehend genannten Säureadditionssalzen der Formel **2** kommt dem Salz, in dem H-X für Salzsäure steht, besondere Bedeutung zu. Nachfolgend wird dieses Säureadditionssalz auch als Telmisartan-hydrochlorid bezeichnet.

Die Verbindungen der Formel **2** sind beispielsweise aus dem aus dem Stand der Technik bekannten 4'-[[2-n-Propyl-4-methyl-6-(1-methylbenzimidazol-2-yl)-benzimidazol-1-yl]-methyl]-biphenyl-2-carbonsäure-*tert*.-butylester (= Telmisartan-tert.-butylester) durch Verseifung in Essigsäure in Gegenwart der Säure H-X erhältlich.

Zur Darstellung des erfindungsgemäßen kristallinen Telmisartan-Natriumsalzes der Formel **1** ausgehend von den Säureadditionssalzen der Formel **2** kann erfindungsgemäß wie folgt vorgegangen werden.

Die Verbindung der Formel **2** wird in einem geeigneten Lösemittel aufgenommen und mit einem geeigneten Natriumsalz versetzt.
Als Lösemittel kommen Wasser und/oder ein geeigneter Alkohol, wie Methanol, Ethanol oder Isopropanol im Gemisch mit einem aprotischen organischen Lösemittel ausgewählt aus der Gruppe bestehend aus Toluol; Chloroform, Dichlormethan, Tetrahydrofuran, Diethylether, Diisopropylether, Methyl-tertiärbutylether, Aceton, Methylisobutylketon, Benzol und Acetonitril in Betracht. Besonders bevorzugt wird als Lösemittel Wasser im Gemisch mit Ethanol oder Isopropanol im Gemisch mit einem aprotischen organischen Lösemittel ausgewählt aus der Gruppe bestehend aus Toluol, Benzol und Methylisobutylketon, besonders bevorzugt Toluol, verwendet. Als besonders geeignet hat sich für diesen Syntheseschritt ein Gemisch aus Wasser, Isopropanol und Toluol erwiesen.
Die Menge an eingesetztem Lösemittel bzw. Lösemittelgemisch ist abhängig von der Menge an eingesetztem Säureadditionssalz **2**. Vorzugsweise werden pro Mol eingesetzter Verbindung **2** etwa 0,3 - 3,5 L, bevorzugt etwa 1 - 2,5 L, besonders bevorzugt etwa 1,5 - 2 L des vorstehend genannten Lösemittels bzw. Lösemittelgemischs verwendet. Gelangt als Lösemittel jenes erfindungsgemäß bevorzugte Lösemittelgemisch zur Anwendung, welches neben Wasser und einem aprotischen organischen Lösemittel ferner einen Alkohol als dritte Lösemittelkomponente enthält, liegen die volumenbezogenen Verhältnisse von Wasser zum aprotischen organischen Lösemittel erfindungsgemäß bevorzugt in einem Bereich von 1:5 bis 1:50 und die von Wasser zum eingesetzten Alkohol in einem Verhältnis von 2:1 bis 1:40. Bevorzugt liegen in einem solchen Lösmittelgemisch die Verhältnisse von Wasser zum aprotischen organischen Lösemittel in einem Bereich von 1:10 bis 1:30, bevorzugt in einem Bereich von 1:15 bis 1:25 und die von Wasser zum eingesetzten Alkohol in einem Verhältnis von 1:1 bis 1:20, bevorzugt in einem Bereich von 1:5 bis 1:15.
Vorzugsweise enthält das vorstehend genannte Lösemittel bzw. Lösemittelgemisch pro Mol **2** etwa 10 bis 100 ml Wasser, bevorzugt etwa 30 bis 80 ml Wasser, besonders bevorzugt etwa 40 bis 70 ml Wasser. Vorzugsweise enthält das eingesetzte Lösemittel bzw. Lösemittelgemisch pro Mol **2** ferner etwa 100 bis 1000 ml Alkohol, bevorzugt etwa 300 bis 800 ml Alkohol, besonders bevorzugt etwa 400 bis 700 ml Alkohol. Schließlich enthält das eingesetzte Lösemittel bzw. Lösemittelgemisch pro Mol **2** vorzugsweise als dritte Lösemittelkomponente etwa 200 bis 2000 ml des vorstehend genannten aprotischen organischen Lösemittels, bevorzugt etwa 600 bis 1600 ml, besonders bevorzugt etwa 800 bis 1400 ml des vorstehend genannten aprotischen organischen Lösemittels.

Als geeignete Natriumsalze kommen für die Umsetzung von **2** zu **1** Natriumhydroxid, Natriumhydrid, Natriumcarbonat, Natriumhydrogencarbonat oder Natriumalkoholate in Betracht. Unter Natriumalkoholaten werden die Natriumsalze verstanden, die mit niederen Alkoholen, vorzugsweise mit Alkoholen ausgewählt aus der Gruppe bestehend aus Methanol, Ethanol, Isopropanol, n-Propanol, tert-Butanol, sec.-Butanol, iso-Butanol, n-Butanol und tert.-Amylalkohol, gebildet werden. Erfindungsgemäß von besonderem Interesse sind Natriumsalze ausgewählt aus der Gruppe bestehend aus Natriumhydroxid, Natriumhydrid, Natriumethanolat und Natriummethanolat, wobei die Natriumalkoholate Natriumethanolat und Natriummethanolat, insbesondere Natriummethanolat für diesen Reaktionsschritt von erfindungsgemäß herausragender Bedeutung ist. Die vorstehend genannten Natriumsalze können der Reaktionsmischung als Feststoffe zugesetzt werden. Im Falle des Natriummethanolats ist die Zugabe in Form methanolischer Lösungen allerdings bevorzugt. Hierbei werden besonders bevorzugt methanolische Lösungen des Natriummethanolats verwendet, die diese in einer Konzentration von wenigstens 10%, besonders bevorzugt von etwa 20-40 % (w/w) enthalten. Beispielsweise kann die methanolische Natriummethanolatlösung mit einer Konzentration von etwa 30 Gew.-% zum Einsatz gelangen.
Die Menge an einzusetzendem Natriumsalz ist naturgemäß abhängig von der Menge an eingesetzter freier Säure Telmisartan. Erfindungsgemäß müssen pro Mol eingesetztes Telmisartan-Säureadditionssalz der Formel **2** wenigstens 2 Mol Natriumsalz zugesetzt werden. Eine Zugabe an Natriumsalz im Überschuß ist erfindungsgemäß ebenfalls möglich.

Gegebenenfalls kann es sinnvoll erscheinen, der vorstehend genannten Reaktionsmischung Aktivkohle zuzusetzen. Beispielsweise kann dies in einer Menge von etwa 5 - 50 g pro Mol eingesetztes **2**, vorzugsweise in einer Menge von etwa 10 - 40 g pro Mol eingesetztes **2** erfolgen.
Nach erfolgter Zugabe des Natriumsalzes sowie gegebenenfalls erfolgter Zugabe von Aktivkohle wird das erhaltene Reaktionsgemisch für einen Zeitraum von etwa 10 Minuten bis 2 Stunden, vorzugsweise für etwa 20-45 Minuten auf eine Temperatur von etwa 50-100°C, vorzugsweise auf etwa 60-90°C, besonders bevorzugt auf etwa 70-80°C erwärmt. Im Zuge dieser Erwärmung kann ein Teil des Lösemittels, vorzugsweise etwa 10-50%, besonders bevorzugt etwa 20-40% der Gesamtlösemittelmenge abdestilliert werden.

Die verbleibende Suspension wird anschließend filtriert, der Filterrückstand gegebenenfalls mit einem der vorstehend genannten aprotischen organischen Lösemittel, vorzugsweise mit jenem aprotischen organischen Lösmittel, welches auch zur Durchführung der Reaktion Verwendung findet, gewaschen.
Das erhaltene Filtrat wird sodann mit einem Lösemittel bzw. Lösemittelgemisch verdünnt. Hierbei wird vorzugsweise eine Gemisch aus Wasser und dem vorstehend genannten aprotischen organischen Lösemittel verwendet. Vorzugsweise werden an dieser Stelle pro Mol ursprünglich eingesetzter Verbindung **2** etwa 10 bis 100 ml Wasser, bevorzugt etwa 30 bis 80 ml Wasser, besonders bevorzugt etwa 40 bis 70 ml Wasser verwendet. An aprotischern organischem Lösemittel werden an dieser Stelle vorzugsweise pro Mol ursprünglich eingesetzter Verbindung **2** 250 bis 3000 ml, bevorzugt etwa 800 bis 2000 ml, besonders bevorzugt etwa 1200 bis 1800 ml verwendet.

Nach Verdünnung wird die erhaltene Mischung bis zum Rückfluß erwärmt. Dabei werden pro Mol ursprünglich eingesetzter Verbindung 2 etwa 1-2 L, vorzugsweise etwa 1200 bis 1800 ml Lösemittel abdestilliert. Nach Abdestillieren des Lösemittels kristallisiert das erfindungsgemäße Telmisartan-Natriumsalz **1** aus. Die erhaltenen Kristalle werden isoliert, gegebenenfalls mit einem der vorstehend genannten aprotischen, organischen Lösemittel gewaschen und abschließend getrocknet.

Kristallines Telmisartan-Natriumsalz, ist auch in Form seiner Solvate oder Hydrate, bevorzugt in Form seiner Hydrate, besonders bevorzugt in Form seines Hemihydrats gemäß vorstehend beschriebener Vorgehensweisen erhältlich.

Aufgrund der pharmazeutischen Wirksamkeit des erfindungsgemäßen kristallinen Telmisartan-Natriumsalzes wird es zur Herstellung eines Arzneimittels verwendet, insbesondere zur Herstellung eines Arzneimittels zur Vorbeugung oder Behandlung von Erkrankungen, in denen die Applikation therapeutisch wirksamer Dosen eines oder mehrerer Angiotensin-II-Antagonisten einen therapeutischen Nutzen entfalten kann. Bevorzugt zielt die vorliegende Erfindung auf die Verwendung von kristallinem Telmisartan-Natriumsalz zur Herstellung eine Arzneimittels zur Vorbeugung oder Behandlung von Erkrankungen ausgewählt aus der Gruppe bestehend aus Hypertonie, Herzinsuffizienz, ischämische periphere Durchblutungsstörungen, myokardiale Ischämie (Angina), Myocard-Infarkt, Herzinsuffizienzprogression nach Myokard-Infarkt, Prävention von cardiovasculären Todesfällen, Schlaganfall, diabetische Neuropathie, diabetische Nephropathie, diabetische Retinopathie, Croscarmellose Natriumsalz (Cellulose carboxymethylether Natriumsalz, quervernetzt), Crospovidone, Natriumstärkeglykolat, Hydroxypropylcellulose (niedrigsubstituiert), Maisstärke, Polyvinylpyrrolidon, Copolymerisaten von Vinylpyrrolidon mit anderen Vinylderivaten (Copovidone), Hydroxypropylcellulose, Hydroxypropylmethylcellulose, Microkristalliner Cellulose oder Stärke, Magnesiumstearat, Natriumstearylfumarat, Talk, Hydroxypropylmethylcellulose, Carboxymethylcellulose, Celluloseacetatphthalat, Polyvinylacetat, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.
In einer pharmazeutischen Zusammensetzung enthaltend als einzigen Wirkstoff das Telmisartan Natriumsalz finden vor allem ein oder mehrere Hilfsstoffe wie Sorbitol, Xylit, Saccharose, Croscarmellose Natriumsalz, Crospovidone, Natriumstärkeglykolat, Hydroxypropylcellulose, Copolymerisaten von Vinylpyrrolidon mit anderen Vinylderivaten (Copovidone), Hydroxypropylcellulose, Hydroxypropylmethylcellulose, Microkristalliner Cellulose oder Natriumstearylfumarat, Hydroxypropylmethylcellulose, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltige Substanzen wie Hartfett oder deren geeignete Gemische Verwendung.
Entsprechende Tabletten erhält man z.B. durch Mischen des oder der Wirkstoffe mit ein oder mehreren Hilfsstoffen und anschließende Verpressung. Beispiele für Hilfsstoffe sind
- inerte Verdünnungsmitteln wie Mannitol, Sorbitol, Xylit, Saccharose, Calciumcarbonat, Calciumphosphat und Lactose;
- Sprengmitteln wie Croscarmellose Natriumsalz (Cellulose carboxymethylether Natriumsalz, quervernetzt), Crospovidone, Natriumstärkeglykolat, Hydroxypropylcellulose (niedrigsubstituiert) und Maisstärke;
- Bindemitteln wie Polyvinylpyrrolidon, Copolymerisaten von Vinylpyrrolidon mit anderen Vinylderivaten (Copovidone), Hydroxypropylcellulose, Hydroxypropylmethylcellulose, Microkristalliner Cellulose oder Stärke;
- Schmiermitteln wie Magnesiumstearat, Natriumstearylfumarat und Talk;
- Mitteln zur Erzielung des Depoteffektes wie Hydroxypropylmethylcellulose, Carboxymethylcellulose, Celluloseacetatphthalat und Polyvinylacetat; und
- Pharmazeutisch zulässige Farbstoffe wie farbige Eisenoxide
Die Tabletten können auch aus mehreren Schichten bestehen.
- Sprengmitteln wie Croscarmellose Natriumsalz (Cellulose carboxymethylether Natriumsalz, quervernetzt), Crospovidone, Natriumstärkeglykolat, Hydroxypropylcellulose (niedrigsubstituiert) und Maisstärke;
- Bindemitteln wie Polyvinylpyrrolidon, Copolymerisaten von Vinylpyrrolidon mit anderen Vinylderivaten (Copovidone), Hydroxypropylcellulose, Hydroxypropylmethylcellulose, Microkristalliner Cellulose oder Stärke;
- Schmiermitteln wie Magnesiumstearat, Natriumstearylfumarat und Talk;
- Mitteln zur Erzielung des Depoteffektes wie Hydroxypropylmethylcellulose, Carboxymethylcellulose, Celluloseacetatphthalat und Polyvinylacetat; und
- Pharmazeutisch zulässige Farbstoffe wie farbige Eisenoxide
Die Tabletten können auch aus mehreren Schichten bestehen.

Die Eigenschaften von Tabletten können manchmal auch dadurch beeinflusst werden, dass einzelne Komponenten und Wirkstoffe vor ihrer Verpressung granuliert und erst dann mit weiteren Hilfsstoffen verpresst werden.

Besonders geeignete Hilfsstoffe für die direkte Verpressung des Telmisartan Natriumsalzes als einziger Wirksubstanz oder zusammen mit dem Diuretikum Hydrochlorothiazid sind Sorbitol und Magnesiumstearat, wobei diese Hilfsstoffe gegebenenfalls durch andere zur Direkttablettierung geeignete Hilfsstoffe wie Mannitol oder Saccharose ersetzt werden können. Um Tabletten mit unterschiedlicher Zusammensetzung an Wirkstoffen auch optisch unterscheiden zu können, ist es vorteilhaft diese Tabletten farblich voneinander zu unterscheiden. Zu diesem Zweck können noch farbgebende Formulierungshilfsstoffe wie farbige Eisenoxide oder andere pharmazeutisch zulässige Farbstoffe vor der Verpressung beigemengt werden.

Ein besonders gutes Löslichkeitsverhalten der Wirkstoffe zeigen Tabletten, bei deren Herstellung das Telmisartan Natriumsalz vor der Verpressung zu Tabletten in einem Trockengranulationsverfahren granuliert wird. Dabei wird das Salz z.B. mit Mannitol, Hydroxypropylcellulose und gegebenenfalls einem fargebenden Hilfsstoff wie rotem Eisenoxid in geeigneten Mischern gemischt, anschließend gesiebt und schließlich einer Trockengranulation z.B. in einem Walzenkompaktor ("Roller Compactor") unterworfen. Die genannten Hilfsstoffe können z.B. durch Hilfsstoffe wie Lactose oder mikrokristalline Cellulose ersetzt werden. Das erhaltene Granulat wird dann gegebenenfalls mit einem weiteren Wirkstoff wie Hydrochlorothiazid sowie mit Hilfsstoffen wie Mannitol, mikrokristalliner Cellulose, Natriumstärkeglykolat, Magnesiumstearat und gegebenenfalls einem fargebenden Hilfsstoff wie rotem Eisenoxid in einem geeigneten Mischer vermischt und schließlich zu Tabletten verpresst. Alternativ können auch Hilfsstoffe wie Lactose oder Croscarmellose Natriumsalz (Cellulose carboxymethylether Natriumsalz, quervernetzt) eingesetzt werden.

Der Gehalt an Telmisartan Natriumsalz beträgt pro Tablette, Dragée oder Kapseln üblicherweise 60-90 mg, 30-60 mg oder 15-30 mg des Salzes. Bevorzugt sind Mengen von 80-85 mg, 40-45 mg oder 20-25 mg. Diese Mengen entsprechen inetwa einem Gehalt von 80 mg, 40 mg bzw. 20 mg freier Säure Telmisartan. Enthalten diese Darreichungsformen auch Hydrochlorothiazid, so liegt dieses in jeder Tablette, Dragée oder Kapsel in einer Menge von 10-15 mg oder 20-30 mg, vorzugsweise 12-13 mg oder 24-26 mg vor.
Verfahren zur Herstellung der genannten pharmazeutischen Zusammensetzungen, besonders jene, bei denen die Wirkstoffe zu Tabletten verpresst werden, sind ebenfalls Gegenstand der vorliegenden Erfindung.

Pharmazeutische Wirkstoffe, die gegebenenfalls zusammen mit dem Telmisartan Natriumsalz in Formulierungen eingearbeitet werden können sind
- Diuretika wie Hydrochlorothiazid;
- Antihypertonica wie
   - ACE-Inhibitoren (z.B. Captopril, Enalapril, Lisinopril, Ramipril und Perindopril) ;
   - Angiotensinrezeptor Antagonisten (z.B. Candesartan, Eprosartan, Irbesartan, Losartan, Telmisartan und Valsartan);
   - Calciumantagonisten (z.B. Nifedipin und Verapamil); oder
   - Alpha- bzw. Betarezeptorblocker (z.B. Ergotamin, Dihydroergotamin, Atenolol, Acebutolol, Metoprolol, Propranolol und Pindolol);
- Antidiabetica wie Nateglinide, Repaglinide und Metformin;
- Thrombozytenaggregationshemmer wie Clopidogrel, Acetylsalicylsäure oder Dipyridamol;
- Vasodilatoren wie Minoxidil;
- Lipid- bzw. Cholesterin-Senker wie Procubol, Sitosterol, MTP Inhibitoren, HMG-CoA-Reduktase-Hemmer wie Lovastatin, Simvastatin und Atorvastatin oder Fibrate.

Das nachfolgende Synthesebeispiel dient der Illustration eines exemplarisch durchgeführter Herstellungsverfahrens für kristallines Telmisartan-Natriumsalz. Es ist lediglich als mögliche, exemplarisch dargestellte Vorgehensweise zu verstehen.

### Synthesebeispiel 1: Darstellung von kristallinem Telmisartan-Natriumsalz ausgehend von Telmisartan:

Als Ausgangsmaterial zur Darstellung von erfindungsgemäßem kristallinem Telmisartan-Natriumsalz kann die freie Säure Telmisartan dienen, die nach im Stand der Technik bekannten Verfahren (z.B. gemäß EP 502314 A1) erhalten werden kann.
In einem geeigneten Reaktionsgefäß werden 154,4 g Telmisartan in 308,8 ml Toluol vorgelegt. Die Suspension wird mit 27,8 g 44,68%iger Natronlauge und 84,9 ml Ethanol versetzt und auf 78°C etwa 30 min erwärmt, der Ansatz anschließend filtriert. Gegebenenfalls kann bei Verbleib größerer Feststoffmengen im Filter mit einem Gemisch aus 61,8 ml Toluol und 15,3 ml Ethanol nachgewaschen werden.

In einem weiteren Reaktionsgefäß werden 463,2 ml Toluol vorgelegt und zum Rückfluß erhitzt. Man tropft in der Siedehitze das gemäß vorstehend beschriebener Vorgehensweise erhältliche Filtrat langsam zu und destilliert gleichzeitig azeotrop ab. Nach Beendigung der Zugabe kann die gegebenenfalls aus der Nachwaschung des Filters erhaltene Lösung ebenfalls zudosiert und auch hier dabei azeotrop abdestilliert werden. Man destilliert bis 103°C und lässt die Suspension auf Raumtemp. abkühlen. Die Kristalle werden abgesaugt, mit 154,4 ml Toluol gewaschen und im Umlufttrockenschrank bei 60°C getrocknet.

Ausbeute: 154,6 g (96%), farblose Kristalle;

| | | | |
|---|---|---|---|
| C₃₃H₂₉N₄O₂Na x 0,5H₂O | ber.: C 72,51 | H 5,72 | N 10,25 |
| | gef.: C 72,57 | H 5,69 | N 10,21 |

### Synthesebeispiel 2: Darstellung von kristallinem Telmisartan-Natriumsalz ausgehend von Telmisartan-hydrochlorid:

### A) Darstellung von Telmisartan-hydrochlorid:

411 g 4'-[[2-n-Propyl-4-methyl-6-(1-methylbenzimidazol-2-yl)-benzimidazol-1-yl]-methyl]-biphenyl-2-carbonsäure-*tert*.-butylester werden in 822 ml Eisessig suspendiert und mit 213 g konzentrierter wässeriger Salzsäure (37%ig) versetzt. Man erhitzt die Mischung zum Rückfluß und destilliert etwa 640 ml Lösemittel ab. Der verbleibende Rückstand wird bei 50-60°C langsam mit etwa 620 ml Wasser versetzt.

Zu dieser Mischung werden 20 g Aktivkohle (z.B. Norit SX 2 Ultra) gegeben und die erhaltene Mischung etwa 10 min bei konstanter Temperatur gerührt. Nach Abfiltrieren wird der Rückstand mit dreimal je 25 ml Eisessig und mit etwa 620 ml Wasser gewaschen. Das erhaltene Filtrat wird erneut auf etwa 50-60°C erwärmt und mit etwa 2 L Wasser versetzt. Nach Rühren über etwa 12 Stunden bei etwa 23°C werden die entstandenen Kristalle abgesaugt und zweimal mit etwa 500 ml Wasser, einmal mit etwa 900 ml Aceton gewaschen und anschließend bei etwa 60°C getrocknet.

Ausbeute: 367 g (92,5%), farblose Kristalle, Schmp.: = 278°C

### B) Darstellung kristallines Telmisartan-Natriumsalz aus Telmisartan-hydrochlorid

Es werden 55,1 g Telmisartan-hydrochlorid in 110,2 ml Toluol, 5,5 ml Wasser, 55,1 ml iso-Propanol aufgenommen und versetzt diese Mischung mit 36,9 g Natriummethylat (30%ig in Methanol) und 2,75 g Aktivkohle (z.B. Sorit SX 2 Ultra). Anschließend wird auf etwa 75°C erwärmt, und bei konstanter Temperatur über etwa 30 min ca. 50 ml Lösungsmittelgemisch abdestilliert. Die erhaltene Suspension wird filtriert und der Rückstand mit etwa 20 ml Toluol gewaschen. Das Filtrat wird mit etwa 5 ml Wasser und etwa 150 ml Toluol versetzt. Die erhaltene Mischung wird zum Rückfluß erhitzt. Dabei werden etwa 150 ml Lösemittelgemisch azeotrop abdestilliert (bei bis zu 102°C). Man lässt eine Stunde bei 100°C durchkristallisieren. Die Kristalle werden abgesaugt, mit etwa 50 ml Toluol gewaschen und bei ca. 60°C getrocknet.

Ausbeute: 53,6 g (99%), farblose Kristalle ;

| | | | |
|---|---|---|---|
| C₃₃H₂₉N₄O₂Na·0,5H₂O | ber.: C 72,51 | H 5,72 | N 10,25 |
| | gef.: C 72,44 | H 5,68 | N 10,20 |

Zur Herstellung eines den Wirkstoff enthaltenden Arzneimittels, insbesondere eines oral applizierbaren Arzneimittels, besonders bevorzugt einer Tablette, kann nach im Stand der Technik bekannten Verfahren vorgegangen werden.
Entsprechende Tabletten können beispielsweise durch Mischen des oder der Wirkstoffe mit bekannten Hilfsstoffen, beispielsweise inerten Verdünnungsmitteln, wie Mannitol, Sorbitol, Xylit, Saccharose, Calciumcarbonat, Calciumphosphat oder Lactose, Sprengmitteln wie Croscarmellose Natriumsalz (Cellulose carboxymethylether Natriumsalz, quervernetzt), Crospovidone, Natriumstärkeglykolat, Hydroxypropylcellulose (niedrigsubstituiert) oder Maisstärke, Bindemitteln wie Polyvinylpyrrolidon, Copolymerisaten von Vinylpyrrolidon mit anderen Vinylderivaten (Copovidone), Hydroxypropylcellulose, Hydroxypropylmethylcellulose, Microkristalliner Cellulose oder Stärke, Schmiermitteln wie Magnesiumstearat, Natriumstearylfumarat oder Talk, und/oder Mitteln zur Erzielung des Depoteffektes wie Hydroxypropylmethylcellulose, Carboxymethylcellulose, Celluloseacetatphthalat, oder Polyvinylacetat erhalten werden. Die Tabletten können auch aus mehreren Schichten bestehen.

Im Folgenden sind einige Beispiele für erfindungsgemäß einsetzbare pharmazeutische Zubereitungen angegeben. Diese dienen lediglich der beispielhaften Erläuterung.

### Formulierungsbeispiel 1: Tablette 1

| Bestandteile: | mg |
|---|---|
| Telmisartan-Natriumsalz | 83,417 |
| Mannitol | 299,083 |
| Mikrokristalline Cellulose | 100,000 |
| Croscarmellose Natriumsalz | 10,000 |
| Magnesiumstearat | 7,500 |
| Gesamt | 500,000 |

### Formulierungsbeispiel 2: Tablette 2

| Bestandteile: | mg |
|---|---|
| Telmisartan-Natriumsalz | 83,417 |
| Sorbitol | 384,083 |
| Polyvidon K25 | 25,000 |
| Magnesiumstearat | 7,500 |
| Gesamt | 500,000 |

### Formulierungsbeispiel 3: Tablette 3

| Bestandteile: | mg |
|---|---|
| Telmisartan-Natriumsalz | 41,708 |
| Mannitol | 149,542 |
| Mikrokristalline Cellulose | 50,000 |
| Croscarmellose Natriumsalz | 5,000 |
| Magnesiumstearat | 3,750 |
| Gesamt | 250,000 |

### Formulierungsbeispiel 4:

Durch direkte Verpressung des Telmisartan Natriumsalzes mit den Hilfsstoffen Sorbitol und Magnesiumstearat werden Tabletten erhalten, deren WirkstoffKonzentration einer Menge von 80 mg, 40 mg bzw. 20 mg freier Säure des Telmisartans entspricht.

**Tablette** enthaltend ein **Äquivalent** von **80 mg** freie Säure Telmisartan:

| **Inhaltstoff** | **mg/Tablette** | **%Tablette** |
|---|---|---|
| Telmisartan Natriumsalz | 83,417 | 17,379 |
| Sorbitol | 389,383 | 81,121 |
| Magnesiumstearat | 7,200 | 1,500 |
| **Total** | **480,000** | **100,000** |

| | | |
|---|---|---|
| Form: oval Abmessungen: 16,2 x 7,9 mm | | |

**Tablette** enthaltend ein **Äquivalent** von **40 mg** freie Säure Telmisartan:

| **Inhaltstoff** | **mg/Tablette** | **%Tablette** |
|---|---|---|
| Telmisartan Natriumsalz | 41,708 | 17,378 |
| Sorbitol | 194,692 | 81,122 |
| Magnesiumstearat | 3,600 | 1,500 |
| **Total** | **240,000** | **100,000** |

| | | |
|---|---|---|
| Form: oval Abmessungen: 12 x 5,9 mm | | |

**Tablette** enthaltend ein **Äquivalent** von **20 mg** freie Säure Telmisartan:

| **Inhaltstoff** | **mg/Tablette** | **%Tablette** |
|---|---|---|
| Telmisartan Natriumsalz | 20,854 | 17,378 |
| Sorbitol | 97,346 | 81,122 |
| Magnesiumstearat | 1,800 | 1,500 |
| **Total** | **120,000** | **100,000** |

| | | |
|---|---|---|
| Form: rund Abmessungen: 7 mm | | |

### Formulierungsbeispiel 5:

Das Telmisartan Natriumsalz wird zunächst mit Mannitol, rotem Eisenoxid, und Hydroxypropylcellulose in einem Intensivmischer ("High-Shear Mixer") vermischt. Anschließend wird durch ein 0,8 mm Sieb gesiebtes Magnesiumstearat beigemengt und die Mischung einer Trockengranulation in einem Walzenkompaktor ("Roller Compactor") unterworfen. Parallel dazu wird Hydrochlorothiazid mit Mannitol, mikrokristalliner Cellulose, Natriumglykolstärke und rotem Eisenoxid in einem Intensivmischer vermischt. Sowohl diese Mischung als auch das Granulat des Telmisartan Natriumsalzes werden durch ein 0,8 mm Sieb gesiebt, miteinander in einem Freifallmischer ("Free Fall Blender") vermischt und schließlich einem abschließenden Mischprozess mit durch ein 0,8 mm Sieb gesiebtem Magnesiumstearat unterworfen. Man erhält eine problemlos verpressbare Zusammensetzung und die daraus hergestellten Tabletten zeigen eine gute Löslichkeit der Wirkstoffe. Diese Zusammensetzung von Wirkstoffen und Hilfsstoffen wird mit einer geeigneten Tablettenpresse (z.B. Korsch EKO oder Fette P1200) verpresst. Es werden Tabletten mit folgender Zusammensetzung hergestellt, wobei die pro Tablette enthaltene Menge Telmisartan Natriumsalz einer Menge von 80 mg der freien Säure des Telmisartans entspricht.

| **Inhaltstoff** | **mg/Tablette** | **%Tablette** |
|---|---|---|
| Telmisartan Natriumsalz | 83,417 | 13,903 |
| Hydrochlorothiazid | 12,500 | 2,083 |
| Mannitol | 336,483 | 56,081 |
| Cellulose microcrystalline | 120,000 | 20,000 |
| Natriumglykolstärke | 30,000 | 5,000 |
| Rotes Eisenoxid | 0,600 | 0,100 |
| Hydroxypropylcellulose | 5,000 | 0,833 |
| Magnesiumstearat | 12,000 | 2,000 |
| **Total** | **600,000** | **100,000** |

Die Zusammensetzung der Tablette kann auch wie folgt beschrieben werden:

| **Inhaltstoff** | **mg/Tablette** | **%/Tablette** | **%/Granulat** |
|---|---|---|---|
| Telmisartan Natriumsalz | 83,417 | 13,903 | 83,417 |
| Mannitol | 10,983 | 1,831 | 10,983 |
| Hydroxypropylcellulose | 5,000 | 0,833 | 5,000 |
| Rotes Eisenoxid | 0,100 | 0,017 | 0,100 |
| Magnesiumstearat | 0,500 | 0,083 | 0,500 |
| **Total** | **100,000** | **16,667** | **100,000** |
| Hydrochlorothiazid | 12,500 | 2,083 | |
| Mannitol | 325,500 | 54,250 | |
| Cellulose microcrystalline | 120,000 | 20,000 | |
| Natriumglykolstärke | 30,000 | 5,000 | |
| Rotes Eisenoxid | 0,500 | 0,083 | |
| Magnesiumstearat | 11,500 | 1,917 | |
| **Total** | **600,000** | **100,000** | |

Die Tabletten weisen folgende Eigenschaften auf:
Abmasse: 16,2 x 7,9 mm (r=5,86 mm)
Gewicht: 598,7 mg ± 0,22 %
Dicke: durchschnittlich 6,16 mm
Bruchfestigkeit: durchschnittlich 145 N
Abrieb: 0,09 %
Zerfallszeit: durchschnittlich 153 s

Unter Rühren (75 rpm) lösen sich 95 ± 3,1 % des Telmisartan Natriumsalzes nach 30 Minuten in 900 ml 0,1 M Phosphatpuffer pH 7,5. 88 ± 3,8 % Hydrochlorothiazid lösen sich nach 30 Minuten in 900 ml 0,1 M HCl (100 rpm).

### Formulierungsbeispiel 6:

Hydrochlorothiazid, Telmisartan Natriumsalz, Sorbitol und rotes Eisenoxid werden in einem Freifallmischer ("Free Fall Blender") gemischt, durch ein 0,8 mm Sieb gesiebt und, nach Zugabe von Magnesium Stearat, in einem Freifallmischer zu einer pulverförmigen Mischung verarbeitet.

Diese Zusammensetzung von Wirkstoffen und Hilfsstoffen wird dann mit einer geeigneten Tablettenpresse (z.B. Korsch EKO oder Fette P1200) zu Tabletten verpresst. Es werden Tabletten mit folgender Zusammensetzung hergestellt, wobei die pro Tablette enthaltene Menge Telmisartan Natriumsalz einer Menge von 80 mg der freien Säure des Telmisartans entspricht.

| **Inhaltstoff** | **mg/Tablette** | **%** |
|---|---|---|
| Telmisartan Natriumsalz | 83,417 | 13,903 |
| Hydrochlorothiazid | 12,500 | 2,083 |
| Sorbitol | 494,483 | 82,414 |
| Rotes Eisenoxid | 0,600 | 0,100 |
| Magnesiumstearat | 9,000 | 1,500 |
| **Total** | **600,000** | **100,000** |

Die Tabletten dreier Chargen weisen folgende Eigenschaften auf:
**Charge 1:**
   Abmasse: 16,2 x 7,9 mm (r=5,86 mm)
   Gewicht: 600,7 mg ± 0,34 %
   Dicke: durchschnittlich 5,96 mm
   Bruchfestigkeit: durchschnittlich 142 N
   Abrieb: 0,12 %
   Zerfallszeit: durchschnittlich 304 s
**Charge 2:**
   Abmasse: 16,2 x 7,9 mm (r=5,86 mm)
   Gewicht: 600,6 mg ± 0,28 %
   Dicke: durchschnittlich 6,11 mm
   Bruchfestigkeit: durchschnittlich 115 N
   Abreib: 0,17 %
   Zerfallszeit: durchschnittlich 331 s
**Charge 3:**
   Abmasse: 16,2 x 7,9 mm (r=5,86 mm)
   Gewicht: 591,1 mg ± 0,56 %
   Dicke: durchschnittlich 5,89 mm
   Bruchfestigkeit: durchschnittlich 116 N
   Abreib: 0,13 %
   Zerfallszeit: durchschnittlich 416 s

Das Telmisartan Natriumsalze der Tabletten der drei Chargen löst sich nach 30 Minuten Rühren (75 rpm) in 900 ml 0,1 M Phosphatpuffer pH 7,5 zu 92 ± 1,5 %, 96 ± 1,8 % bzw. 100 ± 1,0 %. Das Hydrochlorothiazid löste sich nach 30 Minuten in 900 ml 0,1 M HCl (100 rpm) zu 69 ± 6,3 %, 72 ± 2,1 % bzw 78 ± 1,8 %.

## Patentansprüche

1. Pharmazeutische Zusammensetzung enthaltend kristallines Telmisartan Natriumsalz und das Diuretikum Hydrochlorothiazid.

2. Die Zusammensetzung von Anspruch 1 enthaltend ein oder mehrere Formulierungshilfsstoffe ausgewählt aus der Gruppe von Mannitol, Sorbitol, Xylit, Saccharose, Calciumcarbonat, Calciumphosphat, Lactose, Croscarmellose Natriumsalz, Crospovidone, Natriumstärkeglykolat, Hydroxypropylcellulose, Maisstärke, Polyvinylpyrrolidon, Copolymerisaten von Vinylpyrrolidon mit anderen Vinylderivaten (Copovidone), Hydroxypropylcellulose, Hydroxypropylmethylcellulose, Microkristalliner Cellulose oder Stärke, Magnesiumstearat, Natriumstearylfumarat, Talk, Hydroxypropylmethylcellulose, Carboxymethylcellulose, Celluloseacetatphthalat, Polyvinylacetat, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen.

3. Pharmazeutische Zusammensetzung enthaltend kristallines Telmisartan Natriumsalz, gegebenenfalls das Diuretikum Hydrochlorothiazid, und ein oder mehrere Formulierungshilfsstoffe ausgewählt aus der Gruppe von Sorbitol, Xylit, Saccharose, Croscarmellose Natriumsalz, Crospovidone, Natriumstärkeglykolat, Hydroxypropylcellulose, Copolymerisaten von Vinylpyrrolidon mit anderen Vinylderivaten (Copovidone), Hydroxypropylcellulose, Hydroxypropylmethylcellulose, Microkristalliner Cellulose oder Natriumstearylfumarat, Hydroxypropylmethylcellulose, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen.

4. Die Zusammensetzung der Ansprüche 1 bis 3, **dadurch gekennzeichnet dass** das Hydrochlorothiazid in einer Menge von 10-15 mg oder 20-30 mg vorliegt.

5. Die Zusammensetzung der Ansprüche 1 bis 3, **dadurch gekennzeichnet dass** das Natriumsalz des Telmisartans in einer Menge von 60-90 mg vorliegt.

6. Die Zusammensetzung von Anspruch 5, **dadurch gekennzeichnet dass** das Natriumsalz des Telmisartans in einer Menge von 80-85 mg vorliegt.

7. Die Zusammensetzung der Ansprüche 1 bis 3, **dadurch gekennzeichnet dass** das Natriumsalz des Telmisartans in einer Menge von 30-60 mg vorliegt.

8. Die Zusammensetzung von Anspruch 7, **dadurch gekennzeichnet dass** das Natriumsalz des Telmisartans in einer Menge von 40-45 mg vorliegt.

9. Die Zusammensetzung der Ansprüche 6 und 8, **dadurch gekennzeichnet dass** Hydrochlorothiazid in einer Menge von 12-13 mg oder 24-26 mg vorliegt.

10. Die Zusammensetzung von Anspruch 1, **dadurch gekennzeichnet dass** die Wirkstoffe zusammen mit den Hilfsstoffen Sorbitol und Magnesiumstearat direkt zu Tabletten verpresst vorliegen.

11. Die Zusammensetzung von Anspruch 1, **dadurch gekennzeichnet dass** die Wirkstoffe als Verpressung eines Trockengranulats des Telmisartan Natriumsalzes enthaltend die Hilfsstoffe Mannitol, Magnesiumstearat, und Hydroxypropylcellulose mit einer Mischung aus Hydrochlorothiazid, Mannitol, mikrokristalliner Cellulose und Natriumglykolstärke vorliegen.

12. Die Zusammensetzung von Anspruch 1 oder Anspruch 3, **dadurch gekennzeichnet, dass** neben dem Telmisartan-Natriumsalz weitere pharmazeutische Wirkstoffe eingearbeitet sind.

13. Verfahren zur Herstellung der pharmazeutischen Zusammensetzung der Ansprüche 1 bis 3, **dadurch gekennzeichnet dass** die Wirkstoffe mit den Hilfsstoffen zu Tabletten verpresst werden.

## Claims

1. Pharmaceutical composition comprising crystalline telmisartan sodium salt and the diuretic hydrochlorothiazide.

2. The composition according to claim 1 comprising one or more formulation excipients selected from among mannitol, sorbitol, xylitol, saccharose, calcium carbonate, calcium phosphate, lactose, croscarmellose sodium salt, crospovidone, sodium starch glycolate, maize starch, polyvinylpyrrolidone, copolymers of vinylpyrrolidone with other vinyl derivatives (copovidone), hydroxypropylcellulose, hydroxypropylmethylcellulose, microcrystalline cellulose or starch, magnesium stearate, sodium stearylfumarate, talc, carboxymethylcellulose, cellulose acetate phthalate, polyvinyl acetate, water, water/ethanol, water/glycerol, water/sorbitol, water/polyethyleneglycol, propyleneglycol, cetylstearyl alcohol or fatty substances such as hard fat or suitable mixtures thereof.

3. Pharmaceutical composition containing crystalline telmisartan sodium salt, optionally the diuretic hydrochlorothiazide, and one or more formulation excipients selected from among sorbitol, xylitol, saccharose, croscarmellose sodium salt, crospovidone, sodium starch glycolate, copolymers of vinylpyrrolidone with other vinyl derivatives (copovidone), hydroxypropylcellulose, hydroxypropylmethylcellulose, microcrystalline cellulose or sodium stearylfumarate, water, water/ethanol, water/glycerol, water/sorbitol, water/polyethyleneglycol, propyleneglycol, cetylstearylalcohol, carboxymethylcellulose or fatty substances such as hard fat or suitable mixtures thereof.

4. The composition according to claims 1 to 3 **characterised in that** the hydrochlorothiazide is present in an amount of 10-15 mg or 20-30 mg.

5. The composition according to claims 1 to 3, **characterised in that** the sodium salt of telmisartan is present in an amount of 60-90 mg.

6. The composition according to claim 5, **characterised in that** the sodium salt of telmisartan is present in an amount of 80-85 mg.

7. The composition according to claims 1 to 3, **characterised in that** the sodium salt of telmisartan is present in an amount of 30-60 mg.

8. The composition according to claim 7, **characterised in that** the sodium salt of telmisartan is present in an amount of 40-45 mg.

9. The composition according to claims 6 and 8, **characterised in that** hydrochlorothiazide is present in an amount of 12-13 mg or 24-26 mg.

10. The composition according to claim 1, **characterised in that** the active substances are present together with the excipients sorbitol and magnesium stearate, compressed directly into tablets.

11. The composition according to claim 1, **characterised in that** the active substances are present as compressed dry granules of the telmisartan sodium salt comprising the excipients mannitol, magnesium stearate and hydroxypropylcellulose with a mixture of hydrochlorothiazide, mannitol, microcrystalline cellulose and sodium glycol starch.

12. The composition according to claim 1 or claim 3, **characterised in that** in addition to the telmisartan-sodium salt other pharmaceutical active substances are incorporated.

13. Process for preparing the pharmaceutical composition according to claims 1 to 3, **characterised in that** the active substances are compressed with the excipients to form tablets.

## Revendications

1. Composition pharmaceutique contenant du sel de sodium de telmisartan cristallin et le diurétique hydrochlorothiazide.

2. Composition selon la revendication 1 contenant un ou plusieurs adjuvants de formulation choisis dans le groupe : le mannitol, le sorbitol, le xylitol, le saccharose, le carbonate de calcium, le phosphate de calcium, le lactose, le sel de sodium de croscarmellose, la crospovidone, le glycolate d'amidon sodique, l'amidon de maïs, la polyvinylpyrrolidone, les copolymères de vinylpyrrolidone avec d'autres dérivés vinyliques (copovidone), l'hydroxypropylcellulose, l'hydroxypropylméthylcellulose, la cellulose microcristalline ou l'amidon, le stéarate de magnésium, le stéarylfumarate de sodium, le talc, la carboxyméthylcellulose, l'acétophtalate de cellulose, le poly(acétate de vinyle), l'eau, l'eau/éthanol, l'eau/glycérine, l'eau/sorbitol, l'eau/polyéthylèneglycol, le propylèneglycol, l'alcool cétylstéarylique ou les substances contenant des graisses comme la graisse dure ou leurs mélanges appropriés.

3. Composition pharmaceutique contenant du sel de sodium de telmisartan cristallin, éventuellement le diurétique hydrochlorothiazide et un ou plusieurs adjuvants de formulation choisis dans le groupe : le sorbitol, le xylitol, le saccharose, le sel de sodium de croscarmellose, la crospovidone, le glycolate d'amidon sodique, les copolymères de vinylpyrrolidone avec d'autres dérivés vinyliques (copovidone), l'hydroxypropylcellulose, l'hydroxypropylméthylcellulose, la cellulose microcristalline ou le stéarylfumarate de sodium, l'eau, l'eau/éthanol, l'eau/glycérine, l'eau/sorbitol, l'eau/polyéthylèneglycol, le propylèneglycol, l'alcool cétylstéarylique, la carboxyméthylcellulose ou les substances contenant des graisses comme la graisse dure ou leurs mélanges appropriés.

4. Composition selon les revendications 1 à 3 **caractérisée en ce que** l'hydrochlorothiazide est présent en une quantité de 10-15 mg ou 20-30 mg.

5. Composition selon les revendications 1 à 3 **caractérisée en ce que** le sel de sodium du telmisartan est présent en une quantité de 60-90 mg.

6. Composition selon la revendication 5 **caractérisée en ce que** le sel de sodium du telmisartan est présent en une quantité de 80-85 mg.

7. Composition selon les revendications 1 à 3 **caractérisée en ce que** le sel de sodium du telmisartan est présent en une quantité de 30-60 mg.

8. Composition selon la revendication 7 **caractérisée en ce que** le sel de sodium du telmisartan est présent en une quantité de 40-45 mg.

9. Composition selon les revendications 6 à 8 **caractérisée en ce que** l'hydrochlorothiazide est présent en une quantité de 12-13 mg ou 24-26 mg.

10. Composition selon la revendication 1 **caractérisée en ce que** les principes actifs sont présents compressés directement en comprimés en même temps que les adjuvants sorbitol et stéarate de magnésium.

11. Composition selon la revendication 1 **caractérisée en ce que** les principes actifs sont présent sous forme de produit de compression de granulés secs du sel de sodium du telmisartan contenant les adjuvants mannitol, stéarate de magnésium et hydroxypropylcellulose avec un mélange d'hydrochlorothiazide, mannitol, cellulose microcristalline et glycolate d'amidon sodique.

12. Composition selon la revendication 1 ou la revendication 3 **caractérisée en ce que** d'autres principes actifs pharmaceutiques sont incorporés, outre le sel de sodium du telmisartan.

13. Procédé de production de la composition pharmaceutique selon les revendications 1 à 3 **caractérisé en ce que** les principes actifs sont compressés en comprimés avec les adjuvants.
